(19) Europäisches Patentamt
European Patent Office
Office européen des brevets

(11) Publication number: 0 184 855 A2

(12) EUROPEAN PATENT APPLICATION

(21) Application number: 85115920.2

(22) Date of filing: 13.12.85

(51) Int. Cl.⁴: C 07 K 5/00, C 07 K 5/06, A 61 K 37/02

(30) Priority: 14.12.84 US 681516
22.11.85 US 801027

(43) Date of publication of application: 18.06.86 Bulletin 86/25

(84) Designated Contracting States: BE CH DE FR GB IT LI NL SE

(71) Applicant: ABBOTT LABORATORIES, 14th Street and Sheridan Road North St, North Chicago Illinois 60064 (US)

(72) Inventor: Sham, Hing L., 2616 Woodview Court, Waukegan Illinois 60064 (US)

(74) Representative: Modiano, Guido et al, MODIANO, JOSIF, PISANTY & STAUB Modiano & Associati Via Meravigli, 16, I-20123 Milan (IT)

(54) **Resin inhibiting compounds.**

(57) The invention relates to renin inhibiting compounds of the formula

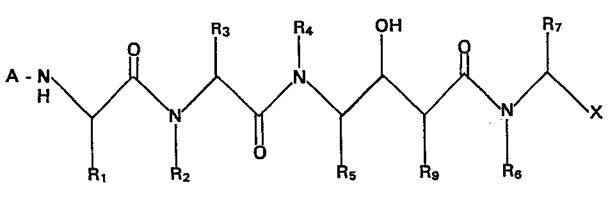

wherein A is an N-protecting group; $R_1$, $R_3$, $R_5$ and $R_7$ are loweralkyl or lipophilic or aromatic amino acid side chains and may be the same or different; $R_2$, $R_4$ and $R_6$ are hydrogen or loweralkyl and may be the same or different; X is hydrogen, loweralkyl or $-Ch_2OR_8$, wherein $R_8$ is hydrogen, loweralkyl or alkaryl; and $R_9$ is loweralkyl, hydroxy, hydroxyalkyl, alkoxy, allyl, alkaryloxy or thioalkyl and pharmaceutically acceptable salts thereof.

EP 0 184 855 A2

## Technical Field

This is a continuation-in-part of U.S. Patent Application, Serial No. 681,516, filed December 14, 1985.

The present invention relates to novel organic compounds which inhibit renin, processes for making such compounds, synthetic intermediates employed in these processes and methods of treating hypertension with such compounds.

## Background Art

Renin is a proteolytic enzyme synthesized and stored principally in a specific part of the kidney called the juxtaglomerular apparatus. Any of three different physiologic circumstances may cause the release of renin into the circulation: (a) a decrease in the blood pressure entering or within the kidney itself; (b) a decrease in the blood volume in the body; or (c) a fall in the concentration of sodium in the distal tubules of the kidney.

When renin is released into the blood from the kidney, the renin-angiotensin system is activated, leading to vasoconstriction and conservation of sodium, both of which result in increased blood pressure. The renin acts on a circulating protein, angiotensinogen, to cleave out a fragment called angiotensin I (AI). AI itself has only slight pharmacologic activity but, after additional cleavage by a second enzyme, angiotensin converting enzyme (ACE), forms the potent molecule angiotensin II (AII). The major pharmacological effects of AII are vasoconstriction and stimulation of the adrenal cortex to release aldosterone, a hormone which causes sodium retention. AII is cleaved by an aminopeptidase to form angiotensin III (AIII), which, compared to AII, is a less potent vasoconstrictor but a more potent inducer of aldosterone release.

Inhibitors of renin have been sought as agents for control of hypertension and as diagnostic agents for identification of cases of hypertension due to renin excess.

With these objectives in mind, the renin-angiotension system has been modulated or manipulated, in the past, with ACE inhibitors. However, ACE acts on several substrates other than angiotensin I (AI), most notably the kinins which cause such undesirable side effects as pain, "leaky" capillaries, prostaglandin release and a variety of behavioral and neurologic effects. Further, ACE inhibition leads to the accumulation of AI. Although AI has much less vasoconstrictor activity than AII, its presence may negate some of the hypotensive effects of the blockade of AII synthesis.

Inhibition of other targets in the renin-angiotensin system such as AII with compounds such as saralasin can block AII activity, but would leave unimpaired and perhaps enhance the hypertensive effects of AIII.

On the other hand, there are no known side effects which result when renin is inhibited from acting on its substrate. Considerable research efforts have thus been carried out to develop useful inhibitors of renin. Past research efforts have been directed to renin antibodies, pepstatin, phospholipids and substrate analogs such as tetrapeptides and octapeptides to tridecapeptides. These inhibitors either demonstrate poor activity in inhibiting renin production or poor specificity for inhibiting renin only. However, Boger et al. have reported that statine-containing peptides possess potent and specific renin-inhibiting activity (<u>Nature</u>, Vol. 303, p. 81, 1983). In addition, Szelke and co-workers have described polypeptide analogs

containing a non-peptide link (Nature, Vol. 299, p. 555, 1982) which also cause potent renin inhibition and show a high specificity for this enzyme.

Even with the discovery of such potent renin inhibitors, it has been observed that the more potent renin inhibitors have larger peptide chains but then poorer oral absorption. Conversely, the smaller peptide chain renin inhibiting compounds have shown good oral absorption, but are less potent. Accordingly, to date no renin inhibiting compounds have been found which are both orally active and potent.

Disclosure of the Invention

In accordance with the present invention, renin inhibiting compounds, which have been found to be both orally active and potent, have the formula

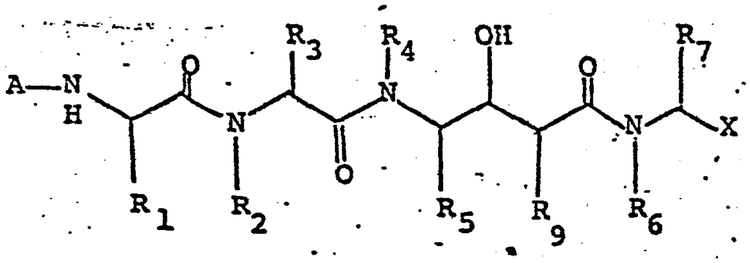

wherein A is an N-protecting group; $R_1$, $R_3$ and $R_5$ and $R_7$ are loweralkyl or lipophilic or aromatic amino acid side chains and may be the same or different; $R_2$, $R_4$, $R_6$ are hydrogen or loweralkyl and may be the same or different; X is hydrogen, loweralkyl or $-CH_2OR_8$ wherein $R_8$ is hydrogen, loweralkyl, or alkaryl; and $R_9$ is loweralkyl, hydroxy, hydroxyalkyl, alkoxy, allyl, alkaryloxy or thioalkyl.

The preferable compounds are when $R_2$, $R_4$, $R_6$ and X are hydrogen, $R_1$ is benzyl, α- or β-naphthylmethyl, $R_3$ is imidazole-4-yl-methyl, methyl, 2-methylthioethyl or methylthiomethyl, $R_5$ is isobutyl, benzyl or cyclohexylmethyl, $R_7$ is isobutyl,

$R_9$ is methoxy, propyl or allyl and A is t-butyloxycarbonyl.

The chiral centers of the compounds of the invention may have either the "R" or "S" configuration but preferably have an "S" configuration.

The term "N-protecting group" as used herein refers to those groups intended to protect the N-terminus against undesirable reactions during synthetic procedures or to prevent the attack of exopeptidases on the final compounds or to increase the solubility of the final compounds and includes but is not limited to acyl, acetyl, pivaloyl, t-butyloxycarbonyl(Boc), carbobenzyloxycarbonyl or benzoyl groups or an L- or D- aminoacyl residue, which may itself be N-protected similarly.

The term "loweralkyl" as used herein refers to straight or branched chain alkyl radicals containing from 1 to 6 carbon atoms including but not limited to methyl, ethyl, n-propyl, iso-propyl, n-butyl, iso-butyl, sec-butyl, 2-methylhexyl, n-pentyl, 1-methylbutyl, 2,2-dimethylbutyl, 2-methylpentyl, 2,2-dimethylpropyl, n-hexyl and the like.

The term "alkaryl" as used herein refers to an unsubstituted or substituted aromatic ring appended to an alkyl radical including but not limited to benzyl, α- and β-naphthylmethyl, halobenzyl and alkoxybenzyl.

The terms "lipophilic or aromatic amino acid side chains" as used herein refers to those amino acid side chains which have an affinity for lipids or have an aromatic ring and include but are not limited to isobutyl, isopropyl, sec-butyl, benzyl, imidazole-4-yl-methyl, p-hydroxybenzyl, α- and β-naphthylmethyl and cyclohexylmethyl. General reference to amino acid side chains in both the description and claims herein is to be taken as

reference to such, whether naturally occurring in proteins or not, and to both D- and L- forms.

The term "hydroxyalkyl" as used herein refers to hydroxy-substituted loweralkyl including but not limited to hydroxymethyl.

The term "alkoxy" as used herein refers to substituents of the formula $-OR_{10}$ wherein $R_{10}$ is loweralkyl and includes but is not limited to methoxy.

The term "alkaryloxy" as used herein refers to substituents of the formula 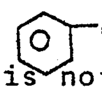 $-(CH_2)_nO-$ wherein n is 1 to 4 and includes but is not limited to benzyloxy.

The term "thioalkyl" as used herein refers to substituents of the formula $-S-R_{11}$ wherein $R_{11}$ is loweralkyl and includes but is not limited to thiomethyl.

The term "allyl" as used herein refers to $H_2C=CH_2CH_2-$.

The term "Ala", "His", "Leu", "Phe" and "Sta" as used herein refers to alamine, histidine, leucine, phenylalamine, and statine, respectively.

The following Examples will serve to illustrate the novel compounds of the present invention and the preparation thereof.

## Example 1

### 4-t-Butyloxycarbonylamino-3-hydroxy-6-methylheptanoic acid ethyl ester

To diisopropylamine (7.7 gm, 0.077 mole) in dry tetrahydrofuran (26 ml) cooled to -20°C under an argon atmosphere was added dropwise n-butyllithium in hexane (1.46 m, 52.4 ml, 0.077 mole). The solution was stirred 15 minutes, the temperature lowered to -78°C and dry ethyl acetate (6.7 g. 0.077 mole) added dropwise while maintaining the temperature below -75°C. The solution was stirred 10 minutes and a precooled (-78°C) tetrahydrofuran solution of Boc-L-leucinal (11 gm., 0.051 mole) was added. After 30 minutes, 2 M HCl (40

ml) was added and the mixture was slowly warmed to 10°C and extracted with ether (3 x 200 ml). The combined ethereal extract was washed with saturated sodium chloride (NaCl) and dried with magnesium sulfate ($MgSO_4$) and filtered. Evaporation of the filtrate in vacuo gave 14 gm of crude product which was purified by flash column chromatography (20% ethylacetate in hexane). Obtained 6 gm of Boc-Sta-OEt.

H' NMR (300 MHz, $CDCl_3$, ppm). 0.93 (d, 6H), 1.27 (t, 3H), 1.3-1.75(m,3H), 1.44(S,9H), 2.50 (m,2H), 3.35(S,1H), 3.63(br m,1H), 4.03(br m1H), 4.18(q,2H), 4.75 (br d,1H).

## Example 2

### 4-t-Butyloxycarbonylamino-3-hydroxy-6-methylheptanoic acid (Boc-Statine)

To 0.8 g of Boc-Sta-OEt in 24 ml of dioxane/water (2:1) was added 120 mg of lithium hydroxide at 0°C. After 10 minutes, the mixture was warmed to room temperature. After 1 hour, the mixture was poured to a 10% solution of potassium bisulfate and extracted with ethyl acetate (3 x 100 ml). The combined organic phase was washed with a satd. NaCl solution and dried with $MgSO_4$ and filtered. Evaporation of the filtrate in vacuo gave 0.7 g of a white solid. Recrystallization from ether/hexane gave a solid with a m.p. of 117°-118°C.

## Example 3

### Boc-Sta amide of benzyl amine

To a solution of 340 mg of Boc-Sta in 25 ml of tetrahydrofuran at -20°C was added 183 µl (1.25 eq.) of N-methylmorpholine, followed by 216 µl (1.25 eg.) of isobutylchloroformate. The solution was stirred for 5 minutes at which time 300 µl (excess) of benzyl amine was added. After 15 minutes at -20°C, the solution was

warmed to 0°C for 30 minutes. The solid was filtered and the filtrate was evaporated in vacuo. The residual oil was dissolved in ethyl acetate (50 ml) and washed with 10 ml of 10% potassium bisulfate solution. The aqueous phase was extracted with ethyl acetate (2 x 50 ml) and the combined ethyl acetate solution was washed with satd. NaCl solution and dried with $MgSO_4$. Evaporation in vacuo gave a colorless oil which upon purification by silca gel column chromatography (5% MeOH /95% $CH_2Cl_2$) gave 370 mg (82%) of pure product as a colorless oil. Mass spectrum: M+ = 364 NMR(60MHz, $CDCl_3$, ppm); 0.95 (d,6H), 1.45 (S,9H), 1.35-1.55 (m,3H), 2.4(d,2H), 3.4-4.4(m,5H) 4.8(brd,1H), 6.9(brd,1H), 7.25(S,5H).

Example 4

Boc-Sta amide of cyclohexylmethyl amine

Using the procedure of Example 3, but replacing benzyl amine with cyclohexylmethyl amine gave the desired compound (85% yield). Mass spectrum: M+ = 370.

Example 5

Boc-Sta amide of isobutylamine

Using the procedure of Example 3, but replacing benzylamine with isobutylamine gave the desired compound (87% yield). Mass spectrum: M+ = 330.

Example 6

Boc-Sta amide of isopentylamine

Using the procedure of Example 3, but replacing benzylamine with isopentylamine gave the desired compound (81% yield). Mass spectrum: M+ = 344.

Example 7

Boc-Sta amide of 2-methylbutylamine

Using the procedure of Example 3, but replacing benzylamine with 2-methylbutylamine gave the desired compound (86% yield). Mass spectrum: M+ = 344.

Example 8

Boc-Sta amide of isoleucinol

Using the procedure of Example 3, but replacing benzylamine with isoleucinol gave the desired compound (85% yield). Mass spectrum: M+ = 374.

Example 9

Boc-Sta amide of leucinol

Using the procedure of Example 3, but replacing benzylamine with leucinol gave the desired compound (84% yield). Mass spectrum: M+ = 374.

Example 10

Boc-Sta amide of valinol

Using the procedure of Example 3, but replacing benzylamine with valinol gave the desired compound (82% yield). Mass spectrum: M+ = 360.

Example 11

Boc-Sta amide of alaninol

Using the procedure of Example 3, but replacing benzylamine with alaninol gave the desired compound (86% yield). Mass spectrum: M+ = 332.

Example 12

Boc-Sta amide of phenylalaninol

Using the procedure of Example 3, but replacing benzylamine with phenylalaninol gave the desired compound (81% yield). Mass spectrum: M+ = 408.

Example 13

Boc-Sta amide of methioninol

Using the procedure of Example 3, but replacing benzylamine with methioninol gave the desired compound (83% yield). Mass spectrum: M+ = 392.

Example 14

Boc-Sta amide of cyclohexylalaninol

Using the procedure of Example 3, but replacing benzylamine with cyclohexylalaninol gave the desired compound (82% yield). Mass spectrum: M+ = 414.

Example 15

Amine hydrochloride of Boc-Sta amide of benzyl amine

Boc-Sta amide of benzylamine (100 mg, 0.27 mmole) was dissolved in 3 ml of 4N HCl and stirred for 10 minutes. The solvent was evaporated in vacuo and the crude product, the amine hydrochloride from deprotection of the N-terminal of Boc-Sta-amide of benzylamine was dried under high vacuum for 12 hours at room temperature. Likewise, the amine hydrochloride of the compounds in Example 4 to Example 14 are prepared by the same procedure.

Example 16

Boc-Phe-His-Sta amide of benzylamine

To the amine hydrochloride of Boc-Sta amide of benzylamine (100 mg. 0.34 mmole) in 4 ml of dimethyl formamide (DMF) was added triethylamine (47 µl, 0.34 mmole). The solution was cooled to 0°C and Boc-Phe-His-OH was added (136 mg, 0.34 mmole), followed by 1-hydroxybenzotriazole (70 mg, 0.51 mmole) and then dicyclohexylcarbodiimide (72 mg, 0.34 mmole). The solution was stirred at 0°C for 8 hours and then at room temperature for 4 hours. The solution was filtered and the solvent was evaporated under vacuum. The residual solid was dissolved in ethyl acetate (50 ml) and washed with saturated sodium bicarbonate and then saturated sodium chloride solution, dried with $MgSO_4$, filtered and the solvent evaporated in vacuo. The crude product was purified by silica gel column (8% MeOH:92%

$CH_2Cl_2$) and 110 mg. (50%) of product was obtained.
m.p. 169°-170°C. Mass spectrum: M+ = 648
analysis calculated for $C_{35}H_{48}N_6O_6$
C 64.79; H 7.46; N 12.95
Found: C 64.56; H 7.40; N 12.81

Example 17

Boc-(α-Naphthyl)-Ala-Ala-Sta amide of benzylamine

To the amine hydrochloride of Boc-Sta amide of benzylamine (100 mg. 0.34 mmole) in THF (3 ml) was added triethylamine (47 μl, 1 equivalent). This solution was added to the mixed anhydride of Boc-(α- Naphthyl)Ala-Ala-OH generated at -20° in the following manner: To Boc-(α- Naphthyl)Ala-Ala-OH (130 mg, 0.34 mmole) in 7 ml of THF at -20°C was added N-methylmorpholine (0.34 mmole), followed by isobutylchloroformate (0.34 mmole). The generation of the mixed anhydride was complete in 5-10 minutes. The reaction mixture was stirred at -20°C for 2 hr. then at 0°C for 30 minutes. It was poured into a 10% solution of potassium bisulfate (40 ml) and extracted with ethylacetate (50 ml x 3). The combined ethyl acetate solution was washed with satd. sodium bicarbonate (40 ml) and then brine (40 ml), dried with $MgSO_4$ and filtered. The solvent was evaporated in vacuo. The residual oil was purified by silica gel column (5% MeOH:95% $CH_2Cl_2$) to give 140 mg of product (65% yield).
m.p. 95°-96°C. Mass spectrum: $(M + H)^+$ = 699

Example 18

Boc-(α-Naphthyl)Ala-Ala-Sta-amide of cyclohexylmethylamine

Using the procedure of Example 17, but using the amine hydrochloride of the Boc-Sta amide of cyclohexylmethylamine gave the desired compound (80% yield). m.p. 109-110°C; Mass spectrum: $(M + H)^+$ = 639.

Example 19

Boc-Phe-His-Sta amide of isobutylamine

Using the procedure of Example 16, but using the amine hydrochloride of Boc-Sta amide of isobutylamine gave the desired compound (60% yield). m.p. 163-164°C; Mass spectrum: $(M + H)^+ = 615$.

Example 20

Boc-(α-Naphthyl)Ala-Ala-Sta-amide of isoleucinol

Using the procedure of Example 17, but using the amine hydrochloride of Boc-Sta amide of isoleucinol gave the desired compound (81% yield). m.p. 181°-182°C; Mass spectrum: $(M + H)^+ = 643$.

Example 21

Boc-(α-Naphthyl)Ala-Ala-Sta-amide of valinol

Using the procedure of Example 17, but using the amine hydrochloride of Boc-Sta amide of valinol gave the desired compound (75% yield). m.p. 185°-186°C; Mass spectrum: $(M + H)^+ = 629$.

Example 22

Boc-Phe-His-Sta-amide of valinol

Using the procedure of Example 16, but using the amine hydrochloride of Boc-Sta amide of valinol gave the desired product (59% yield). m.p. 109°-110°C; Mass spectrum: $(M + H)^+ = 645$.

Example 23

Boc-(α-Naphthyl)Ala-Ala-Sta amide of alaninol

Using the procedure of Example 17, but using the amine hydrochloride of the Boc-Sta amide of alaninol gave the desired product (76% yield). m.p. 176°-177°C; Mass spectrum: $(M + H)^+ = 601$.

Example 24

Boc-(α-Naphthyl)Ala-Ala-Sta amide of leucinol

Using the procedure of Example 17, but using the amine hydrochloride of the Boc-Sta amide of leucinol gave the desired product (72% yield). m.p. 191°-192°C; Mass spectrum: $(M + H)^+ = 643$.

Example 25

Boc-(α-Naphthyl)Ala-Ala-Sta amide of cyclohexylalaninol

Using the procedure of Example 17, but using the amine hydrochloride of the Boc-Sta amide of cyclohexylphenylalaninol gave the desired product (74% yield). m.p. 186°-187°C; Mass spectrum: $(M + H)^+ =$ 683.

Example 26

Boc-(α-Naphthyl)Ala-Ala-Sta amide of methioninol

Using the procedure of Example 17, but using the amine hydrochloride of the Boc-Sta amide of methioninol gave the desired product (76% yield). m.p. 183°-184°C; Mass spectrum: $(M + H)^+ = 661$.

Example 27

Boc-Sta amide of phenethyl amine

Using the procedure of Example 3, but replacing benzylamine with phenethylamine gave the desired compound (72% yield). Mass spectrum: M+ = 380.

Example 28

Boc-Sta amide of 4-amino-1-benzylpiperidine

Using the procedure of Example 3, but replacing benzylamine with 4-amino-1-benzylpiperidine gave the desired compound (70% yield). Mass spectrum: M+ = 447.

Example 29

Boc-Phe-His-Sta-amide of phenethylamine

Using the procedure of Example 16, but using the amine hydrochloride of Boc-Sta amide of phenethylamine gave the desired compound (61% yield). m.p. 188-191°C; Mass spectrum: $(M + H)^+$ = 663.

Example 30

Boc-Phe-His-Sta-amide of 4-amino-1-benzylpiperidine

Using the procedure of Example 16, but using the amine hydrochloride of Boc-Sta amide of 4-amino-1-benzylpiperidine gave the desired compound (52% yield). m.p. 118°-119°C; Mass spectrum: $(M + H)^+$ = 732.

Example 31

Boc-His-Sta amide of 2-methylbutylamine

Using the procedure of Example 16, but using the amine hydrochloride of Boc-Sta amide of 2-methylbutylamine and replacing Boc-Phe-His with Boc-His-OH gave the desired compound (60% yield). m.p. 115°-116°C; Mass spectrum: M+ = 681.

Example 32

t-Butylacetyl-Phe-His-Sta amide of 2-methylbutylamine

Using the procedure of 17, but using the amine hydrochloride of Example 31 and replacing Boc(α-Naphthyl)-Ala-Ala-OH with t-Butylacetyl-Phe-OH gave the desired compound (45% yield). m.p. 186°-188°C; Mass spectrum: $(M + H)^+$ = 627.

Example 33

Boc-p-iodo-Phe-His-Sta amide of 2-methylbutylamine

Using the procedure of Example 17, but using the amine hydrochloride of Example 31 and replacing Boc(α-Naphthyl)-Ala-Ala with Boc-p-iodo-Phe-OH gave the

desired compound (65% yield). m.p. 213°C (decomp.); Mass spectrum: $(M + H)^+ = 755$.

Example 34

4-t-Butyloxycarbonylamino-5-phenyl-3-hydroxypentanoic acid ethyl ester (Boc-F-Sta ethyl ester)

Using the procedure of Example 1, but replacing Boc-L-leucinal with Boc-L-phenylalaninal gave the desired compound (52% yield). Mass spectrum: M+ = 337.

Example 35

4-t-Butyloxycarbonylamino-5-phenyl-3-hydroxypentanoic acid. (Boc-F-Sta)

Using the procedure of Example 2 but using the compound in Example 34 gave the desired compound (95% yield). m.p. 88°C.

Example 36

Boc-F-Sta amide of isopentylamine

Using the procedure of example 3, but replacing Boc-Sta with Boc-F-Sta and replacing benzylamine with isopentylamine gave the desired compound (75% yield). m.p. 157°-158°C; Mass spectrum: M+ = 378

Example 37

Boc-Phe-His-F-Sta amide of isopentylamine

Using the procedure of Example 16, but using the amine hydrochloride of Boc-F-Sta amide of isopentylamine gave the desired compound (51% yield). m.p. 201°-202°C; Mass spectrum: $(M + H)^+ = 663$.

Example 38

4-t-Butyloxycarbonylamino-3-hydroxy-5-cyclohexylpentanoic acid ethyl ester (Boc-ACHPA ethyl ester)

Using the procedure of Example 1, but replacing Boc-Leucinal with Boc-Cyclohexylalaninal gave the

desired compound in 40% yield. Mass spectrum: $M^+ = 343$.

Example 39

4-t-Butyloxycabonylamino-3-hydroxy-5-cyclohexylpentanoic acid (Boc-ACHPA)

Using the procedure of Example 2, but using the compound from Example 38 gave the desired compound (100% yield). Mass spectrum: $M^+ = 315$.

Example 40

Boc-ACHPA amide of isopentylamine

Using the procedure of Example 3, but replacing Boc-Sta with Boc-ACHPA and benzyl amine with isopentylamine gave the desired compound (70% yield). Mass spectrum: $M^+ = 384$.

Example 41

Boc-Phe-His-ACHPA amide of isopentylamine

Using the procedure of Example 16, but using the amine hydrochloride of Boc-ACHPA amide of isopentylamine gave the desired compound (42% yield). m.p. 108°-110°C. Mass spectrum: (M + H) = 669.

Example 42

4-t-Butyloxycarbonylamino-2-benzyloxy-3-hydroxy-5-cyclohexylpentanoic acid methyl ester

Using the procedure of Example 1, but replacing Boc-leucinal with Boc-cyclohexylalaninal, and replacing ethyl acetate with benzyloxymethyl acetate gave the desired compound in 14.5% yield. Mass spectrum: $M^+ = 435$.

Example 43

4-t-Butyloxycarbonylamino-2-benzyloxy-3-hydroxy-5-cyclohexylpentanoic acid

Using the procedure of Example 2, but using the compound from Example 42 gave the desired compound (100% yield). Mass spectrum: $M^+ = 421$.

Example 44

4-Boc-amino-2-benzyloxy-3-hydroxy-5-cyclohexylpentanoic acid amide of 2-methylbutylamine

Using the procedure of Example 3, but replacing Boc-Sta with the compound in Example 43 and benzyl amine with 2-methylbutylamine gave the desired compound (72% yield). Mass spectrum: $M^+ = 490$.

Example 45

4-Boc-amino-2,3-dihydroxy-5-cyclohexylpentanoic acid amide of 2-methylbutylamine

A solution of 400 mg of the compound in Example 44 in 20 mL of methanol with 200 mg of palladium black added was stirred vigorously under 3 atmosphere of hydrogen for 17 hrs. The catalyst was filtered off and the solution was concentrated in vacuo. The crude product was purified by silica gel chromatography to give 203 mg (62% yield) of desired product. Mass spectrum: $M^+ = 400$.

Example 46

Amine hydrochloride of 4-Boc-amino-2-benzyloxy-3-hydroxy-5-cyclohexyl-pentanoic acid amide of 2-methylbutylamine

Using the procedure of Example 15, but replacing Boc-Sta amide of benzylamine with the compound in Example 44 gave the desired product (100% yield).

Example 47

Amine hydrochloride of 4-Boc-amino-2,3-dihydroxy-5-cyclohexylpentanoic acid amide of 2-methylbutylamine

Using the procedure in Example 15, but replacing Boc-Sta amide of benzylamine with the compound in Example 45 gave the desired product (100% yield).

## Example 48

Boc-Phe-Alaninyl-4-amino-2-benzyloxy-3-hydroxy-5-cyclohexylpentanoic acid amide of 2-methylbutylamine

Using the procedure of Example 17, but replacing Boc-(α-Naphthyl)Ala-Ala-OH with Boc-Phe-Ala-OH and using the amine hydrochloride in Example 46 gave the desired product (59% yield). Mass spectrum: $(M + H)^+ = 709$.

## Example 49

Boc-Phe-Histidinyl-4-amino-2-benzyloxy-3-hydroxy-5-cyclohexylpentanoic acid amide of 2-methylbutylamine

Using the procedure of Example 16, but using the amine hydrochloride in Example 46 gave the desired product (40% yield). Mass spectrum: $(M + H)^+ = 776$.

## Example 50

Boc-Phe-Alaninyl-4-amino-2,3-dihydroxy-5-cyclohexylpentanoic acid amide of 2-methylbutylamine

Using the procedure of Example 17, but replacing Boc-( α-Naphthyl)Ala-Ala-OH with Boc-Phe-Ala-OH and using the amine hydrochloride in Example 47 gave the desired product. Mass spectrum: $(M + H)^+ = 619$.

## Example 51

Boc-Phe-Histidinyl-4-amino-2,3-dihydroxy-5-cyclohexylpentanoic acid amide of 2-methylbutylamine

Using the procedure in Example 17, but using the amine hydrochloride in Example 47 gave the desired product (35% yield). Mass spectrum: $(M + H)^+ = 685$.

Example 52

4-t-Butyloxycarbonylamino-2-methoxy-3-hydroxy-5-cyclohexylpentanoic acid ethyl ester

Using the procedure of Example 1, but replacing Boc-Leucinal with Z-cyclohexylalaninal, and replacing ethyl acetate with methoxy ethyl acetate gave the desired product (33% yield). Mass spectrum: $M^+ = 407$.

Example 53

4-t-Butyloxycarbonylamino-2-methoxy-3-hydroxy-5-cyclohexylpentanoic acid

Using the procedure of Example 2, but using the compound from Example 52 gave the desired product (100% yield). Mass spectrum: $M^+ = 379$.

Example 54

4-Boc-amino-2-methoxy-3-hydroxy-5-cyclohexylpentanoic acid acid amide of 2-methylbutylamine

Using the procedure of Example 3, but replacing Boc-Sta with the compound in Example 53 and benzyl amine with 2-methylbutylamine gave the desired product (80% yield). Mass spectrum: $(M + H)^+ = 449$.

Example 55

Amine hydrochloride of 4-Boc-amino-2-methoxy-3-hydroxy-5-cyclohexylpentanoic acid amide of 2-methylbutylamine

Using the procedure of Example 15, but replacing Boc-Sta amide of benzyl amine with the compound in Example 54 gave the desired compound (100% yield).

Example 56

Boc-Phe-Histidinyl-4-amino-2-methoxy-3-hydroxy-5-cyclohexylpentanoic acid amide of 2-methylbutylamine

Using the procedure of Example 16, but using the amine hydrochloride in Example 55 gave the desired product (35% yield). Mass spectrum: $(M + H)^+ = 699$.

Example 57

4-t-Butyloxycarbonylamino-2-allyl-3-hydroxy-5-cyclohexylpentanoic acid ethyl ester

Using the procedure of Example 1, but replacing Boc-Leucinal with Boc-cyclohexylalaninal, and replacing ethyl acetate with ethyl-4-pentenoate gave the desired product in 55% yield. Mass spectrum: $M^+ = 383$.

Example 58

4-t-Butyloxycarbonylamino-2-allyl-3-hydroxy-5-cyclohexylpentanoic acid

Using the procedure of Example 2, but using the compound from Example 57 gave the desired compound (100% yield). Mass spectrum: $M^+ = 355$.

Example 59

4-Boc-amino-2-allyl-3-hydroxy-5-cyclohexylpentanoic acid amide of 2-methylbutylamine

Using the procedure of Example 16, but replacing Boc-Phe-His-OH with the compound in Example 58 and using 2-methylbutylamine instead of an amine hydrochloride gave the desired product (50% yield). Mass spectrum: $M^+ = 424$.

Example 60

Amine hydrochloride of 4-t-Boc-amino-2-allyl-3-hydroxy-5-cyclohexylpentanoic acid amide of 2-methylbutylamine

Using the procedure of Example 15, but replacing Boc-Sta amide of benzylamine with the compound in Example 59 gave the desired compound (100% yield).

Example 61

Boc-Phe-Histidinyl-4-amino-2-allyl-3-hydroxy-5-cyclohexylpentanoic acid amide of 2-methylbutylamine

Using the procedure of Example 16, but using the amine hydrochloride in Example 60 gave the desired product (40% yield). Mass spectrum: $(M + H)^+ = 709$.

Example 62

Boc-Phe-Histidinyl-4-amino-2-propyl-3-hydroxy-5-cyclohexylpentanoic acid amide of 2-methylbutylamine

A solution of 11 mg of the compound of Example 61 in methanol with 3 mg of 10% palladium on charcoal added was stirred vigorously under hydrogen atmosphere for 2 hours at room temperature. The catalyst was filtered off and the filtrate concentrated to give a colorless oil. Purification by silica gel column chromatography gave 11 mg of pure product (100% yield). Mass spectrum: $(M + H)^+ = 711$.

Example 63

Boc-Phe-Methioninyl-4-amino-2-methoxy-3-hydroxy-5-cyclohexylpentanoic acid amide of 2-methylbutylamine

Using the procedure of Example 17, but replacing Boc-(α-Naphthyl)-Ala-Ala-OH with Boc-Phe-Met-OH and using the amine hydrochloride in Example 55 gave the desired compound (80% yield). Mass spectrum: $(M + H)^+ = 693$.

Example 64

Boc-Phe-S-methylcysteinyl-4-amino-2-methoxy-3-hydroxy-5-cyclohexlpentanoic acid amide of 2-methylbutylamine

Using the procedure of Example 17, but replacing Boc-(α-Naphthyl)-Ala-Ala-OH with Boc-Phe-S-methylcysteine and using the amine hydrochloride in Example 55 gave the desired compound (63% yield). Mass spectrum: $(M + H)^+ - 679$.

Example 65

Boc-Phe-Alaninyl-4-amino-2-allyl-3-hydroxy-5-cyclohexylpentanoic acid amide of 2-methylbutylamine

Using the procedure of Example 17, but replacing Boc-(α-Naphthyl)-Ala-Ala-OH with Boc-Phe-Ala-OH and using the amine hydrochloride in

Example 60 gave the desired compound (56% yield). Mass spectrum: $(M + H)^+ = 643$.

Example 66

Boc-Phe-S-methylcysteinyl-4-amino-2-allyl-3-hydroxy-5-cyclohexylpentanoic acid amide of 2-methylbutylamine

Using the procedure of Example 17, but replacing Boc-(α-Naphthyl)-Ala-Ala-OH with Boc-Phe-S-methylcysteine and using the amine hydrochloride in Example 60 gave the desired compound (52% yield). Mass spectrum: $(M + H)^+ = 689$.

The compounds of the present invention can be used in the form of salts derived from inorganic or organic acids. Included among such salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptonate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Also, the basic nitrogen-containing groups can be quaternized with such agents as loweralkyl halides, such as methyl, ethyl,, propyl and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others. Water or oil-soluble or dispersible products are thereby obtained.

The novel compounds of the present invention possess an excellent degree of activity and specificity in treating renin-associated hypertension in a host. The ability of the compounds of the invention to inhibit human renal renin can be demonstrated _in vitro_ by reacting a selected compound at varied concentrations with human renal renin, free from acid proteolytic activity, and with human renin substrate (angiotensinogen) at 37°C and pH 6.0. At the end of the incubation, the amount of angiotensin I formed is measured by radioimmunoassay and the molar concentration required to cause 50% inhibition, expressed as the $IC_{50}$, is calculated. When tested in accordance with the foregoing procedure, the compounds of the invention demonstrated $IC_{50}$'s in the range of $10^{-5}$ to $10^{-10}$ M as seen in Table I.

Total daily dose administered to a host in single or divided doses may be in amounts, for example, from 0.001 to 10 mg/kg body weight daily and more usually 0.01 to 1 mg. Dosage unit compositions may contain such amounts or submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The compounds of the present invention may be administered orally, parenterally, by inhalation spray, rectally or topically in dosage unit formulations containing conventional nontoxic pharmaceutically acceptable carriers, adjuvants and vehicles as desired. The term parenteral as used herein includes subcutaneous injections, intravenous, intramuscular, intrasternal injection or infusion techniques.

Injectable preparation, for example, sterile injectable aqueous or oleagenous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectibles.

Suppositories for rectal administration of the drug can be prepared by mixing the drug with a suitable nonirritating excipient such as cocoa butter and polyethylene glycols which are solid at ordinary temperatures but liquid at the rectal temperature and will therefor melt in the rectum and release the drug.

Solid dosage forms for oral administration may include capsules, tablets, pills, powders and granules. In such solid dosage forms, the active compound may be admixed with at least one inert diluent such as sucrose, lactose or starch. Such dosage forms may also comprise,

as is normal practice, additional substances other than inert diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration may include pharmaceutically acceptable emulsions, solutions, suspensions, syrups and elixirs containing inert diluents commonly used in the art, such as water. Such compositions may also comprise adjuvants, such as wetting agents, emulsifying and suspending agents, and sweetening, flavoring and perfuming agents.

The foregoing is merely illustrative of the invention and is not intended to limit the invention to the disclosed compounds. Variations and changes which are obvious to one skilled in the art are intended to be within the scope and nature of the invention which are defined in the appended claims.

TABLE I

| Example | Activity $-IC_{50}$ (M) |
|---|---|
| 17 | $4 \times 10^{-7}$ |
| 16 | $7 \times 10^{-7}$ |
| 18 | $7 \times 10^{-7}$ |
| 19 | $5 \times 10^{-7}$ |
| 20 | $5 \times 10^{-8}$ |
| 21 | $2 \times 10^{-7}$ |
| 22 | $1 \times 10^{-6}$ |
| 23 | $1.5 \times 10^{-7}$ |
| 24 | $7 \times 10^{-8}$ |
| 25 | $4 \times 10^{-7}$ |
| 26 | $1 \times 10^{-7}$ |
| 29 | $8 \times 10^{-7}$ |
| 30 | 46% at $10^{-5}$M |
| 32 | $1 \times 10^{-7}$ |
| 37 | $8 \times 10^{-8}$ |
| 33 | $1 \times 10^{-6}$ |
| 41 | $5 \times 10^{-9}$ |
| 48 | $6 \times 10^{-9}$ |
| 49 | $5 \times 10^{-9}$ |
| 50 | $1.5 \times 10^{-8}$ |
| 51 | $7 \times 10^{-9}$ |
| 56 | $3 \times 10^{-8}$ |
| 61 | $1.5 \times 10^{-9}$ |
| 62 | $3 \times 10^{-9}$ |
| 63 | $1 \times 10^{-9}$ |
| 64 | $7 \times 10^{-10}$ |
| 65 | $1.5 \times 10^{-9}$ |
| 66 | $1 \times 10^{-9}$ |

WE CLAIM:

1. A renin inhibiting compound of the formula

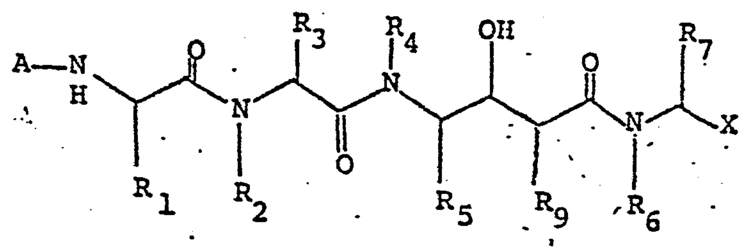

wherein A is an N-protecting group; $R_1$, $R_3$, $R_5$ and $R_7$ are loweralkyl or lipophilic or aromatic amino acid side chains and may be the same or different; $R_2$, $R_4$ and $R_6$ are hydrogen or loweralkyl and may be the same or different; X is hydrogen, loweralkyl or $-CH_2OR_8$, wherein $R_8$ is hydrogen, loweralkyl or alkaryl; and $R_9$ is loweralkyl, hydroxy, hydroxyalkyl, alkoxy, allyl, alkaryloxy or thioalkyl and pharmaceutically acceptable salts thereof.

2. The compound of claim 1 wherein $R_2$, $R_4$, and $R_6$ are hydrogen.

3. The compound of claim 2 wherein X is hydrogen, loweralkyl, or $-CH_2OH$.

4. The compound of claim 2 wherein A is t-butyloxycarbonyl.

5. The compound of claim 2 wherein $R_9$ is methoxy, propyl or allyl.

6. The compound of claim 2 wherein $R_1$ is benzyl or α- or β-naphthylmethyl; $R_5$ is isobutyl; cyclohexylmethyl or benzyl; $R_3$ is imidazole-4-yl-methyl, methylthioethyl, methylthiomethyl or methyl; and $R_7$ is loweralkyl or imidazole-4-yl-methyl.

7. The compound of claim 6 wherein $R_1$ is benzyl; $R_5$ is cyclohexylmethyl; $R_7$ is isobutyl; $R_9$ is methoxy, propyl or allyl; X is hydrogen and A is t-butyloxycarbonyl.

8. A pharmaceutical composition for treating renin-associated hypertension, comprising a pharmaceutical carrier and a therapeutically effective amount of the compound of claim 1.

9. A method of treating hypertension comprising administering to a host in need of such treatment a therapeutically effective amount of a compound of claim 1.

1275j